# EUROPEAN PATENT APPLICATION

(11) **EP 3 090 712 A1**
(43) Date of publication of application: **09.11.2016**
(21) Application number: 15166616.1
(22) Date of filing: 06.05.2015
(51) Int. Cl.: A61F 13/51, D04H 1/4391, D04H 1/544, D04H 3/018, D04H 3/14

(54) **NON-WOVEN FABRIC AND PROCESS FOR FORMING THE SAME**

(71) Applicant: Fitesa Germany GmbH, 31224 Peine (DE)
(72) Inventor: Novarino, Elena, 30539 Hannover (DE); Siebner, Harald, 38122 Braunschweig (DE); Hartl, Helmut, 38124 Braunschweig (DE)
(74) Representative: van Wijk, Alexander Pieter

(57) **Abstract**

The present invention relates to a nonwoven fabric comprising a nonwoven web which is formed from a plurality of trilobal fibers, wherein the lobes of the trilobal fibers each have a ratio of length to width in the range of from 1-4. The present invention also relates to the use of the nonwoven fabric in a closure system in an absorbent article or in a reinforcement layer of an absorbent article. In addition, the present invention provides an absorbent article comprising the nonwoven fabric.

## Description

### FIELD OF THE INVENTION

The present invention relates to a nonwoven fabric, an absorbent article comprising the non-woven fabric, the use of the nonwoven fabric in a closure system in an absorbent article or in a reinforcement layer of an absorbent article, and an absorbent article comprising the nonwoven fabric.

### BACKGROUND OF THE INVENTION

Nonwoven fabrics are widely applied in disposable absorbent articles for personal care or hygiene, such as diapers, feminine care articles, incontinence articles and children's training pants. A particular category amongst such absorbent articles are adult incontinence articles which comprise an absorbent pad, a frontal ear and a waist belt that needs to be attached around the waist of the adult wearer. The materials of which waist belts and frontal ear components in adult incontinence articles are made need to meet a number of criteria to ensure that wearing comfort is established for the adult wearer. One critical factor is that these materials need to have sufficient stiffness to avoid excessive wrinkling and twisting of the absorbent article. Another critical factor is that the materials display sufficient softness to avoid skin problems associated with cutting and abrasion. Moreover, the appearance of softness is of great importance in such articles since it reassures the wearer that the article will be experienced as comfortable.

Object of the present invention is to provide a nonwoven fabric from which components for absorbent articles, in particular adult incontinence articles, can be made which provide sufficient stiffness and at the same time establish improved wearer comfort.

### SUMMARY OF THE INVENTION

It has now been found that this can be established when use is made of nonwoven fabrics comprising a nonwoven web which is formed from a particular type of fibers.

Accordingly, the present invention relates to nonwoven fabric comprising a nonwoven web which is formed from a plurality of trilobal fibers, wherein the lobes of the trilobal fibers each have a ratio of length to width (L/W) in the range of from 1-4.

A major advantage of the present invention resides in the fact that the present nonwoven fabric enables the manufacture of adult incontinence articles which display enough strength to fix the article properly around the waist of the wearer, whereas at the same time the wearer will experience improved wear comfort. Moreover, the present nonwoven fabrics make it possible to produce articles in which the waist belt and the outer side of the article are made of the same fiber materials, contributing to the experience of wearer comfort. Another major advantage of the present nonwoven fabrics is that they provide higher stiffness at lower basis weights. Further, due to the use of the particular fibers no further measures are needed to stiffen the nonwoven fabrics. This means for instance that the nonwoven fabrics do not need a separate meltblown layer or the addition of polyesters to provide sufficient support. Hence, the nonwoven fabrics in accordance with the present invention bring about considerable cost reductions when compared with the use of conventional nonwoven fabrics in absorbent articles, in particular adult incontinence articles. Moreover, the recycling of these articles is very much improved since no separate meltblown layers or polyesters are needed to provide sufficient stiffness to the nonwoven fabrics.

### DETAILED DESCRIPTION OF THE INVENTION

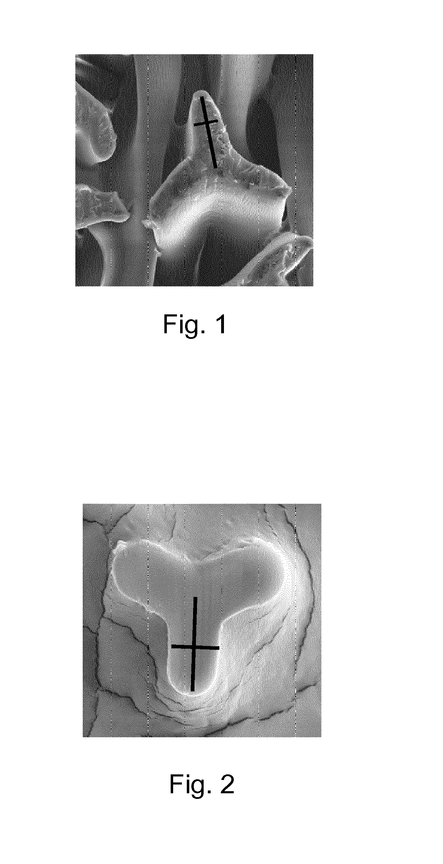

In accordance with the present invention the nonwoven fabric comprises a nonwoven web which is formed from a plurality of trilobal fibers, wherein the lobes of the trilobal fibers each have a ratio of length to width (L/W) in the range of from 1-4. Preferably, the lobes of the trilobal fibers each have a ratio of length to width in the range of from 2.5-4. In the context of the present invention the length of a lobe means the distance between the end point of a lobe and the center of the core of the trilobal fiber (see also Figures 1 and 2).

Suitably, in accordance with the present invention the trilobal fibers are made from thermoplastic polymers. The nonwoven webs particularly suitable are made from fibers of thermoplastic polymers such as polyolefins, polyesters, ethylene copolymers, propylene copolymers, butene copolymers, and combinations thereof, but may also comprise natural fibers such as wood, cotton, or rayon in combination with thermoplastic fibers. The nonwoven web may also be a composite made up of a mixture of two or more different fibers or a mixture of fibers and particles.

The fibers are suitably joined by bonding to form a coherent web structure. Suitable bonding techniques include, but are not limited to, chemical bonding and thermal bonding, for example thermal calendering or bonding by a hot gas stream.

A wide range of suitable polyolefins can be used in the present invention. Suitable examples include polyethylene, polypropylene, copolymers of ethylene and butene, hexene, or octene, copolymers of polypropylene and ethylene. The polyolefins may comprise a homopolymer or a copolymer such as propylene-α-olefins copolymers. In particularly, the latter copolymers can attractively be used in the present invention. Preferred are polyolefin materials that comprise a propylene-α-olefin copolymer and a propylene homopolymer.

The melt flow rate (MFR) of the polyolefin material is suitably less than 90 dg/min. The MFR is determined using ASTM test method D1238, 2.16 kg. Preferably, the MFR of the polyolefin material is in the range of from 15-50 dg/min, more preferably in the range of from 15-35 dg/min.

Preferably, the fibers are formed from polyethylene or polypropylene homopolymers, co-polymers thereof, blends of polyethylene and polypropylene, a polyester, co-polymers of polyesters and/or blends of polyesters.

Suitably use is made of a propylene-based or ethylene-based homopolymer or a copolymer. In the case of propylene-based polymers the polymers may comprise comonomer-derived units selected from ethylene and C4-C10 α-olefins. In the case of ethylene-based polymers the polymers may comprise comonomer-derived units selected from C3-C10 α-olefins. Suitable examples of polyolefin materials include propylene homopolymers, ethylene homopolymers, propylene copolymers and ethylene copolymers such as linear low density polyethylene (LLDPE), high density polyethylene (HDPE), and low density polyethylene (LDPE).

Suitable polyesters can be aliphatic polyesters such as, e.g. polylactic acid, or aromatic polyesters such as polyethylene terephthalate (PET) and poly(trimethylene terephthalate (PTT).

Besides additives already contained in the employed polymers, addition of further additives is possible to provide additional properties to the fibers. Suitable further additives include thermal stabilizers, light stabilizers, slip additives, waxes, and additives to make the fabrics either hydrophilic or hydrophobic. The addition of filler materials can sometimes also be of advantage. Suitable filler materials include organic and inorganic filler materials. Suitable examples of inorganic filler materials include minerals such as calcium carbonate, metals such as aluminium and stainless steel. Suitable examples of organic filler materials include sugar-based polymers.The trilobal fibers to be used in accordance with the present invention can suitable be single component or multicomponent fibers such as bicomponent fibers. In case use is made of bicomponent fibers the lobes will be made of one type of polymer and the core to which the lobes are attached will be made of another type of polymer. Most preferred are core-sheath bicomponent fibers comprising polyethylene and polypropylene, but any other combination of other suitable polymers is possible as well, for example combinations of polyesters with polyolefins. The bicomponent fibers may contain different types of polypropylene. More preferably, the bicomponent fiber has a core of a polypropylene which has a higher melting point and lobes of a polypropylene which has a lower melting point. In another embodiment, the bicomponent fiber comprises two polypropylenes that differ in melt temperature or melt flow.

The fibers can be made according to spinning technologies known in the art. Most conveniently employed are spunbond processes, from which the nonwoven fabrics can directly be formed.

Spunbond fibers are generally produced by extruding a molten polymer through a large spinneret having several thousand holes per linear meter or from banks of smaller spinnerets, for example, containing as few as 40 holes. After exiting the spinneret, the molten fibers are quenched by a cross-flow air quench system, then pulled away from the spinneret and attenuated by high speed air. Lay-down of the filaments to create a nonwoven layer occurs on a permeable transport belt. Spunbond fibers are generally continuous and range in fiber diameter between ca. 10-100 µm.

The non-woven fabric in accordance with the invention can additionally be treated to add specific properties. Most common are topical treatments to make the fabric either hydrophilic or to make it hydrophobic. Most common is the treatment of the fabric with either hydrophilic surfactants or with a fluorocarbon or a silicon material. In the context of the present invention a surface of a non-woven fabric or non-woven web is "hydrophilic" when the contact angle of water disposed on that surface is less than about 90 and a surface is "hydrophobic" when the contact angle of water disposed on that surface is greater than or equal to 90.

Preferably, the nonwoven fabrics in accordance with the invention are hydrophobic non-woven fabrics.

The nonwoven fabric according to the invention can consist of only one type of fibers or fiber layers, e.g. a spunbond layer, but it suitably can comprise additional fiber layers which may be different. Suitable multi-layer fabrics, for example, may include one or more spunbond layers (S) and one or more meltblown layers (M), such as SMS, SMMS, SSMMS, etc. adhered to form a nonwoven fabric according to the present invention. Usually, these multilayer fabrics are made in one step on a single line with multiple beams, which generally encompass a combination of spunbond and meltblown beams. In some cases it might be advantageous or technically necessary to make a multiple layer according to the invention in two or more separate steps. A major advantage of the present invention is however that the nonwoven fabrics of the present invention do not require additional fiber layers such as meltblown layers to increase their stiffness.

Combination of spunbond layers with natural fibers is possible as well. Preferably, the additional nonwoven webs to be used in accordance with the present invention are made of meltblown fibers.

Suitably, the nonwoven web according to the present invention has a tensile strength according WSP 110.4 in MD (Machine Direction) in the range of from 1-4 N per gram basis weight, preferably in the range of from 1.2-3.5 N per gram basis weight, and more preferably in the range of from 1.3-3.0 N per gram basis weight. Non-woven webs with such tensile strengths provide non-woven articles with a high tensile strength.

Suitably, the nonwoven web has a basis weight of at least 8 gsm (g/m²) and less than 40 gsm. Preferably, the non-woven web has a basis weight in the range of from 10-35 gsm, more preferably in the range of 10-30 gsm, even more preferably in the range of 10-25 gsm, and most preferably in the range of 10-20 gsm.

This WSP test method is an internationally acknowledged test method in the non-woven's industry, as the skilled person will understand.

The present nonwoven fabrics are suitably made from fibers that have a weight in the range of from 1-6 dtex, preferably in the range of from 1.5-5 dtex, and more preferably in the range of from 1.8-4 dtex.

Suitably, the nonwoven webs to be used in accordance with the present invention comprise at most 25 wt% of meltblown fibers, based on total weight of the non-woven web. Preferably, the non-woven webs comprises at most 20 wt% of meltblown fibers, based on the total weight of the non-woven web.

Suitably, the present nonwoven webs contain spunbond fibers only, not mixtures of spunbond fibers and another type of fibers.

The nonwoven fabrics in accordance with the present invention comprise a nonwoven web which has a side which is provided with a pattern of bonded areas and non-bonded areas, and wherein the surface of the bonded areas is in the range of 10-32 % of the total surface of the side and the surface of the non-bonded area is in the range of from 68-90 % of the total surface of the side.

Therefore, the present invention also relates to a nonwoven fabric comprising a nonwoven web which is formed from a plurality of trilobal fibers, wherein the lobes of the trilobal fibers each have a ratio of length to width in the range of from 1-4, wherein the nonwoven web which has a side which is provided with a pattern of bonded areas and non-bonded areas, and wherein the surface of the bonded areas is in the range of 10-32 % of the total surface of the side and the surface of the non-bonded area is in the range of from 68-90 % of the total surface of the side. Suitably, the nonwoven web which has a side which is provided with a first pattern of individualized bonded areas and a second pattern of non-bonded areas, and wherein the surface of the bonded areas is in the range of 10-32 % of the total surface of the side and the surface of the non-bonded area is in the range of from 68-90 % of the total surface of the side.

The present invention therefore also provides a nonwoven fabric comprising a nonwoven web which is formed from a plurality of trilobal fibers, wherein the lobes of the trilobal fibers each have a ratio of length to width in the range of from 1-4, wherein the nonwoven web which has a side which is provided with a first pattern of individualized bonded areas which define a second pattern of non-bonded areas, and wherein the surface of the bonded areas is in the range of 10-32 % of the total surface of the side and the surface of the non-bonded area is in the range of from 68-90 % of the total surface of the side.

The trilobal fibers to be used according to the present invention allow a more open bond pattern (lower bond area) when compared with the denser bond patterns that are obtained with for instance round fibers, whilst achieving the same degree of required stiffness. In addition, the higher stiffness of the present fibers does advantageously not directly translate into a lower softness of the fabric. To the contrary, the more open bond pattern that is established with the present invention, allows for higher bulkiness and fluffiness, which results in an increased perceived softness.

The high surface of the non-bonded areas to be used according to the present invention provides an attractively high softness. Moreover, the large unbonded areas allow for the fiber to bulk up and increase the bulkiness of the fabric. This is perceived as an even higher softness from both visual and the tactile perspective. Preferably, the surface of the non-bonded area is at least 68% and less than 90% of the total surface area of the side. Preferably, the surface of the non-bonded area is in the range of from 75-85% of the total surface area of the side.

The surface of the bonded area is preferably more than 10% and at most 32%, more preferably in the range of from 15-25 % of the total surface area of the side.

In a very preferred embodiment, a first pattern of individualized bonded areas defines a second pattern of non-bonded areas which non-bonded areas have a hexagonal type of shape.

Preferably, the side of the non-woven fabric is only provided with the first pattern and the second patterns, meaning that no further pattern of bonded or non-bonded areas is provided on the side of the non-woven fabric.

Preferably, the individualized bonded areas have a non-linear shape. In the context of the present application a non-linear shape is defined as a shape which is not linear as such or does not contain one or more linear parts.

Suitably, the non-bonded areas have the shape of a regular or an irregular hexagon in which one or more sides have a different length. Preferably, the non-bonded areas have a regular hexagonal shape.

Suitably, the non-bonded areas have a surface in the range of from 20-50 mm², preferably in the range of from 22-45 mm², and more preferably in the range of from 23-40 mm².

The individualized bonded areas suitably have a symmetrical shape such as a circle, diamond, rectangle, square, oval, triangle, heart, moon star, hexagonal, octagonal or another polygon shape. Preferably, the bonded areas have a circle or hexagonal shape. More preferably, the bonded areas have a circle shape.

The bonded areas suitably have a maximum width in the range of from 0.7-1.5 mm, preferably in the range of from 0.75-1.25 mm, and more preferably in the range of from 0.8-1.2 mm.

Suitably, the bonded areas have a surface in the range of from 0.38-1.77 mm², preferably in the range of from 0.44-1.22 mm², and more preferably in the range of from 0.50-1.13 mm². The discrete non-bonded areas suitably have a depth in the range of from 0.4-1.5 mm, preferably in the range of from 0.4-0.9 mm, more preferably in the range of from 0.4-0.8 mm, and most preferably in the range of from 0.5-0.7 mm.

Suitably, an even number of bonded areas defines an individual non-bonded area. Preferably, the individual non-bonded areas are defined by 6, 12, 18 or 24 individualized bonded areas, more preferably 12, 18 or 24 individualized bonded areas, and most preferably by 12 individualized bonded areas.

The present invention also relates to an absorbent article comprising a nonwoven fabric according to the present invention. Suitably, the absorbent article according to the present invention is a disposable hygiene absorbent article selected from the group consisting of incontinence articles, diapers, wipes and fem-care articles. Suitable disposable hygiene absorbent articles according to the present invention include those selected from the group consisting of baby diapers, pull-ups, training pants, hygiene closure systems, adult incontinence briefs and diapers. Preferably, the absorbent article in accordance with the present invention is an adult incontinence article.

The nonwoven fabric according to the present invention can suitably be part of a top sheet, back sheet, landing zone and/or a waist belt, wing or a frontal ear. Preferably, the present nonwoven fabric is part of a closure system in adult incontinence article, preferably a waist belt, wing or frontal ear.

The present invention also relates to the use of the nonwoven fabric according to the present invention in a closure system in an absorbent article. Preferably, the closure system comprises a waist belt, frontal ear or wing.

The present invention further relates to the use of the nonwoven fabric according to the present invention in a reinforcement layer of an absorbent article.

In Figures 1 and 2, pictures are shown of trilobal fibers which are used in accordance with the present invention. In Figure 1, a trilobal fiber is shown with a ratio of length to width of 1:3, whereas in Figure 2 a trilobal fiber is shown with a ratio of length to width of 1:2.

### Examples

In these Examples a comparison is made between nonwoven fabrics made from round fibers and nonwoven fabrics in accordance of the present invention which is made from trilobal fibers. The stiffness of the two respective nonwoven fabrics is compared, whereby the stiffness is expressed in terms of bending length. The bending length is measured according to WSP 90.5.

### Example 1

In this Example, the surface of the bonded areas for both the nonwoven fabrics made from round fibers and trilobal fibers was 10.9 %, based on the total surface of one of the sides of the nonwoven fabrics. Further, in both cases the fiber titer was 2.2 dtex, and both nonwoven fabrics had a basis weight of 13 gsm. In Table 1, the stiffness in terms of bending length and the thickness of the fibers is indicated. The thickness of the round fibers is defined as the diameter of the fiber, whereas the thickness of the trilobal fibers is defined as the distance between the end points of two respective lobes.

**Table 1**

| **13 gsm** | Round fiber fabric | Trilobal fiber fabric |
|---|---|---|
| Bending length | 17 mm | 25 mm |
| Thickness nonwoven fabric (bulk) | 300 µm | 597 µm |
| Fiber thickness | 16 µm | 26 µm |

### Example 2

In this Example, the surface of the bonded areas for both the nonwoven fabrics made from round fibers and trilobal fibers had a bonded area of 18.1 %, based on the total surface of one of the sides of the nonwoven fabrics. Further, in both cases the fiber titer was 2.2 dtex, and both nonwoven fabrics had a basis weight of 17 gsm. In Table 2, the stiffness in terms of bending length and the thickness of the fibers is indicated. The thickness of the round fibers is defined as the diameter of the fiber, whereas the thickness of the trilobal fibers is defined as twice the distance between the end point of a single lobe and the center of the core of the fiber.

**Table 2**

| **17 gsm** | Round fiber fabric | Trilobal fiber fabric |
|---|---|---|
| Bending length | 25 mm | 33 mm |
| Thickness nonwoven fabric (bulk) | 317 µm | 397 µm |
| Fiber thickness | 16 µm | 26 µm |

From Tables 1 and 2 it will be clear that the, nonwoven fabrics made from round fibers need a higher basis weight as well as higher bond area to establish a bending length of 25mm when compared with nonwoven made from triblobal fibers. Actualy, to achieve a bending length of 25 mm, a round fiber fabric needs 17 gsm and a bonded area of 18.1% while a trilobal fabric achieves same bending length with already 13 gsm and a bonding area of 10.9%. Moreover, the nonwoven fabric made from trilobal fibers had a bulkiness in terms of fabric thickness which was approximately twice as high when compared with the bulkiness of the nonwoven fabric made from the round fibers. In light of these findings it will be clear that the nonwoven fabrics according to the present invention constitute a major improvement over conventional nonwoven fabrics which are made from round fibers.

## Claims

1. A nonwoven fabric comprising a nonwoven web which is formed from a plurality of trilobal fibers, wherein the lobes of the trilobal fibers each have a ratio of length to width in the range of from 1-4.

2. The nonwoven fabric according to claim 1, wherein the lobes of the trilobal fibers each have a ratio of length to width in the range of from 2.5-4.

3. The nonwoven according to claim 1 or 2, wherein the trilobal fibers are made from thermoplastic polymers.

4. The nonwoven according to claim 3, wherein the thermoplastic polymers comprise polypropylene and/or polyethylene.

5. The nonwoven fabric according to any one of claims 1-4, wherein the trilobal fibers have a weight in the range of from 1.5-5 dtex.

6. The nonwoven fabric according to any one of claims 1-5, wherein the nonwoven web has a basis weight in the range of from 10-35 gsm.

7. The nonwoven fabric according to any one of claims 1-6, wherein the nonwoven web has a side which is provided with a pattern of bonded areas and non-bonded areas, and wherein the surface of the bonded areas is in the range of 10-32 % of the total surface of the side and the surface of the non-bonded area is in the range of from 68-90 % of the total surface of the side.

8. The nonwoven fabric according to any one of claims 1-7, wherein the nonwoven web has a side which is provided with a first pattern of individualized bonded areas that define a second pattern of non-bonded areas.

9. Use of the nonwoven fabric according to any one of claims 1-8 in a closure system in an absorbent article.

10. Use according to claim 9, wherein closure system comprises a waist belt, frontal ear or wing.

11. Use of the nonwoven fabric according to any one of claims 1-8 in a reinforcement layer of an absorbent article.

12. An absorbent article comprising a nonwoven fabric according to any one of claims 1-8.

13. An absorbent article according to claim 12, wherein the article is an adult incontinence article.
